# EUROPEAN PATENT SPECIFICATION

(11) **EP 2 639 298 B1**
(45) Date of publication and mention of the grant of the patent: **04.01.2017**
(21) Application number: 11840247.8
(22) Date of filing: 01.11.2011
(51) Int. Cl.: C12N 5/076, A01K 67/02, A01N 1/02

(54) **THE METHOD FOR PREPARING HUMAN SEMEN FOR IN VITRO FERTILIZATION OR FOR ARTIFICIAL INSEMINATION**
VERFAHREN ZUR VORBEREITUNG VON MENSCHLICHEN SPERMA ZUR IN-VITRO-FERTILISATION ODER ZUR KÜNSTLICHEN BEFRUCHTUNG
PROCÉDÉ DE PERPARATION DES SPERMATOZOIDES HUMAINS CONGELÉS POUR FERTILISATION IN VITRO OU POUR INSÉMINATION ARTIFICIELLE

(30) Priority: 09.11.2010 JP 2010251342
(43) Date of publication of application: 18.09.2013
(73) Proprietor: Hiroshima University, Hiroshima 739-8511 (JP); Renia Medical Group, Tokyo 204-0021 (JP)
(72) Inventor: SHIMADA, Masayuki, Higashi-Hiroshima-shi Hiroshima 739-8528 (JP); OKAZAKI, Tetsuji, Higashi-Hiroshima-shi Hiroshima 739-8528 (JP); FUJITA, Yoko, Tokyo 178-0063 (JP)
(74) Representative: Epping - Hermann - Fischer
(86) International application number: PCT/JP2011/075219
(87) International publication number: WO 2012/063687

(56) References cited:
- EP-A1- 2 443 920
- US-A1- 2003 215 782
- Masayuki Shimada: "Developing a Method of Swine Artificial Insemination Using Frozen-Thawed Spermatozoa －How to Create the Next-Generation System of Pig-Breeding, Supporting not only Japan's but also all of world's Pig Industry (original in Japanese)", Research@HU > Research NOW>Edition 26, 25 June 2010 (2010-06-25), pages 1-5, XP055122765, Hiroshima University Retrieved from the Internet: URL:http://www.hiroshima-u.ac.jp/upload/0/ kenkyu/now/no26/shiryou.pdf [retrieved on 2014-06-11]
- CHI-HYUN LEE ET AL: "Characterization of in-Vitro Spermicidal Activity of Chelating Agent against Human Sperm", JOURNAL OF PHARMACEUTICAL SCIENCES, vol. 85, no. 6, 1 June 1996 (1996-06-01), pages 649-654, XP055122788,
- H. KUSAKABE ET AL: "Mouse and human spermatozoa can be freeze-dried without damaging their chromosomes", HUMAN REPRODUCTION, vol. 23, no. 2, 1 December 2007 (2007-12-01), pages 233-239, XP055122794, ISSN: 0268-1161, DOI: 10.1093/humrep/dem252
- "Quinn's Advantage Sperm Freeze", , 1 March 2003 (2003-03-01), pages 1-2, XP055122944, Retrieved from the Internet: URL:http://www.kbbiosystem.com/pdf/DFU/DFU -8022.pdf [retrieved on 2014-06-12]
- "QUINN'S Sperm Washing Medium", , 1 March 2003 (2003-03-01), page 1, XP055122949, Retrieved from the Internet: URL:http://www.kbbiosystem.com/pdf/DFU/DFU -1005,1006.pdf [retrieved on 2014-06-12]
- MASAYUKI SHIMADA ET AL.: 'The effects of seminal plasma in pig artificial insemination using cryopreserved spermatozoa: the development of chemical defined seminal plasma for pig artificial insemination' ALL ABOUT SWINE February 2010, pages 10 - 15, XP008169444
- TETSUJI OKAZAKI ET AL.: 'Seishi Hogozai to Chakusho Sokushinzai o Mochiita Buta Toketsu Seishi ni yoru Jinko Juseiho no Kaihatsu' LIVESTOCK TECHNOLOGY 01 December 2009, pages 8 - 11, XP008149015
- TETSUJI OKAZAKI ET AL.: 'Toketsu Seieki o Mochiita AI no Jitsuyoka o Mezashite 2) -Seishi Hogozai to Chakusho Sokushinzai o Tenka shita Seishi Kishakueki no Kaihatsu' YOTONKAI vol. 44, no. 13, 01 December 2009, pages 38 - 41, XP008169587
- CURRY,M.R.: 'Cryopreservation of mammalian semen' METHODS MOL.BIOL. vol. 368, 2007, pages 303 - 311, XP008169404
- OKAZAKI,T. ET AL.: 'The addition of calcium ion chelator, EGTA to thawing solution improves fertilizing ability in frozen-thawed boar sperm' ANIM.SCI.J. vol. 82, no. 3, June 2011, pages 412 - 419, XP055087419

## Description

The present invention relates to a method for preparing semen for in vitro fertilization or semen for artificial insemination in humans.

### Background Art

It is said that, in Japan, one out of four couples faces an infertility problem due to the influence of the trend toward delayed marriage and the like. Therefore, infertility treatments such as artificial insemination, as well as advanced assisted reproductive technologies such as in vitro fertilization and microinsemination are being increasingly carried out.

In artificial insemination and in vitro fertilization, the husband also needs to visit the clinic where the infertility treatment is carried out. However, because the number of spermatozoa collected varies from day to day depending on the physical condition, and motility of the spermatozoa collected varies, the treatment is impossible in many cases. Further, in some cases, it is impossible for the husband to visit the clinic because of urgent business, and the treatment is cancelled. Further, in cases of a husband who needs to be treated with an anticancer agent, carrying out the anticancer agent therapy increases the risk of occurrence of oligospermia, and hence the patient sometimes wishes collection and preservation of his spermatozoa before the anticancer agent therapy.

Because of such a background, cryopreservation of spermatozoa is an important technique also in humans. However, at present, in artificial insemination using spermatozoa cryopreserved by a known technique, the pregnancy rate is extremely low, so that frozen semen is used mainly in cases of microinsemination (ICSI) wherein direct injection into an ovum is carried out by micromanipulation.

At present, many types of solutions for freezing human spermatozoa are commercially available. Representative examples of the solutions include a commercially available liquid supplemented with chicken yolk and glycerol (sperm freezing medium, IS Japan). Spermatozoa frozen using the commercially available liquid are thawed and cultured using an embryo culture medium, and the resulting spermatozoa are used for microinsemination, in vitro fertilization, artificial insemination or the like.

Further, in the livestock industry, a method wherein frozen spermatozoa are thawed with a diluent before artificial insemination is disclosed (Non Patent Literature 1, Patent Literature 1).

### Citation List

### Non Patent Literature

Non Patent Literature 1: Tetsuji Okazaki, Shuji Yoshida, Hisanori Teshima and Masayuki Shimada. Development of a fully synthesized thawing liquid (artificial seminal plasma) to be used for artificial insemination with pig frozen-thawed spermatozoa. Abstracts of the 112th meeting of the Japanese Society of Animal Science, IYS-02. Date of presentation, March 28, 2010.

### Patent Literature

Patent Literature 1: International Publication WO 2010/147194

### Summary of Invention

### Technical Problem

Spermatozoa prepared by preparing frozen spermatozoa using the above commercially available liquid and thawing/culturing the spermatozoa in an embryo culture medium do not necessarily have high sperm motility. Because of such low sperm motility after thawing, use of the spermatozoa in in vitro fertilization (excluding microinsemination) or artificial insemination leads to a low pregnancy rate, which is problematic.

Further, in Non Patent Literature 1 and Patent Literature 1, the dilution liquid for frozen spermatozoa is intended for use in polytocous animals such as pig, and the dilution liquid is not directly applicable to human frozen spermatozoa. For example, Shimada et al. developed a method of Swine artificial insemination using frozen-thawed spermatozoa (conference presentation on June 24, 2010, edition 26, pages 1-5, Hiroshima University). US 2003/0215782 A1 discloses a composition for freezing or freeze-drying spermatozoa. Lee et al. evaluated the effect of EDTA on sperm motility (J Pharm Sci, 1996, 85, 649-654). Kusakabe et al. report that chromosome damage in freeze-dried mouse spermatozoa can be alleviated when spermatozoa are kept in an EGTA solution for several days before freeze-drying (Hum Reprod, 2008, 23, 233-239). EP 2443 920 A1 discloses a sperm diluent solution and a method for artificial insemination using the same. Quinn's Advantage Sperm Freeze and Sperm Washing Medium (registered trademarks) information and directions for use disclose a method and composition for in vitro procedures involving freezing and washing of spermatozoa.

The present invention was made in view of the above problems, and aims to provide a diluent for human frozen spermatozoa that allows suppression of a decrease in sperm motility after thawing human frozen spermatozoa, a method for diluting human frozen spermatozoa, and a method for preparing semen for in vitro fertilization or semen for artificial insemination in humans.

### Solution to Problem

In the method for preparing semen for in vitro fertilization or semen for artificial insemination in humans of the present invention, human frozen spermatozoa are thawed and mixed with a diluent for human frozen spermatozoa, and spermatozoa are then separated from the diluent for human frozen spermatozoa, the diluent for human frozen spermatozoa, comprising a chelating agent that forms a complex with a calcium ion, and having a pH of 6.9 to 7.1, said chelating agent comprises EGTA and EDTA..

Further, the spermatozoa are preferably separated within 15 minutes after the start of thawing.

Further, the separated spermatozoa are preferably cultured in an embryo culture medium.

Further, human frozen spermatozoa prepared by separating human semen into spermatozoa and seminal plasma, dehydrating the separated spermatozoa by hyperosmotic treatment, and freezing the dehydrated spermatozoa are preferably used.

Further, a hyperosmotic liquid with an osmotic pressure of more than 300 mOsm/kg and less than 500 mOsm/kg is preferably used.

Further, human frozen spermatozoa prepared by adding glycerol and performing glycerol equilibration under conditions where the glycerol concentration is higher than 2% by volume and lower than 5% by volume, followed by freezing, are preferably used.

### Advantageous Effects of Invention

The diluent for human frozen spermatozoa in the methods of the present invention contains a chelating agent that forms a complex with a calcium ion, and has a pH of 6.9 to 7.1, said chelating agent comprises EGTA and EDTA. Since calcium ions form complexes with the chelating agent, the action of a large amount of calcium ions on spermatozoa during thawing and immediately after thawing of human frozen spermatozoa is suppressed. Therefore, vigorous movement of spermatozoa by the action of calcium ions, which causes death of the spermatozoa, during thawing and immediately after thawing is suppressed, and sperm motility is maintained for a long time after thawing. Therefore, improvement of the pregnancy rate is expected in artificial insemination, in vitro fertilization and the like.

In the method for preparing semen for in vitro fertilization or semen for artificial insemination in humans of the present invention, human frozen spermatozoa are thawed and mixed with the diluent for human frozen spermatozoa described above. By separating the thawed spermatozoa from the diluent for human frozen spermatozoa within a predetermined period of time after the start of thawing of the human frozen spermatozoa, semen for in vitro fertilization or semen for artificial insemination in humans is obtained. Suppressing the drastic action of calcium ions on the spermatozoa during thawing and immediately after thawing, followed by separating the thawed spermatozoa from the diluent for human frozen spermatozoa, allows supply of calcium necessary for the action of spermatozoa. Since, in the thus obtained semen for in vitro fertilization or semen for artificial insemination in humans, the activity of spermatozoa is maintained for a long time, it is possible to use the semen not only in in vitro fertilization but also in artificial insemination.

### Brief Description of Drawings

FIG. 1 is a graph showing the results of measurement of sperm motility in Comparatives examples 1 and 1a (using Medium 199 as diluent);
FIG. 2 is a graph showing the results of measurement of sperm motility in Comparatives examples 2 and 2a (using diluent for pig frozen spermatozoa pH=7.4);
FIG. 3 is a graph showing the results of measurement of sperm motility in Example 2 and Comparative Example 3;
FIG. 4 is a graph showing the results of measurement of sperm motility that was carried out 6 hours after thawing of human frozen spermatozoa in Example 3 and Comparative Example 4;
FIG. 5 is a graph showing the results of measurement of sperm motility that was carried out 24 hours after thawing of human frozen spermatozoa in Example 3 and Comparative Example 4; and
FIG. 6 is a photograph showing the results of detection of tyrosine phosphorylation bands in Example 4 and Comparative Example 5.

### Description of Embodiments

The method for preparing semen for in vitro fertilization or semen for artificial insemination in humans, of the present mode are described below in detail.

The diluent for human frozen spermatozoa used in the methods of the present invention is a liquid for thawing human frozen spermatozoa and quickly mixing the spermatozoa. The diluent for human frozen spermatozoa contains the later-mentioned basic diluent in which a mixture of EGTA and EDTA that forms a complex with a calcium ion is contained. Since the diluent for human frozen spermatozoa contains a chelating agent, phosphorylation of proteins in the spermatozoa that occurs due to involvement of calcium ions upon thawing of human frozen spermatozoa is suppressed, and the dysfunction is thereby suppressed.

More specifically, the middle piece of a spermatozoon contains mitochondria, and the mitochondria produce energy (ATP) to be used for movement of the spermatozoon. When calcium ions act on the mitochondria (more specifically, an enzyme for production of ATP), production of ATP is activated, causing vigorous movement of the spermatozoon.

When calcium ions act on human frozen spermatozoa during thawing or immediately after thawing of the spermatozoa, the spermatozoa are drastically activated. Spermatozoa need to have high activity upon fertilization, but drastic activation of spermatozoa during thawing or immediately after thawing of the spermatozoa does not allow the activity of the spermatozoa to be maintained until fertilization, causing death of the spermatozoa. Therefore, it is impossible to expect improvement of the pregnancy rate in in vitro fertilization, artificial insemination and the like.

In the diluent for human frozen spermatozoa used in the methods of the present invention the chelating agent forms complexes with calcium ions during thawing and immediately after thawing of human frozen spermatozoa, and the action of the calcium ions on mitochondria of the spermatozoa is thereby suppressed. Therefore, the death of spermatozoa due to drastic activation is suppressed, and survival of the spermatozoa is secured.

As a reference, the diluent for human frozen spermatozoa has a pH of not less than 6.75 and less than 7.4. As described in the later-mentioned Examples, the sperm motility after thawing is higher than the sperm motility observed in cases where human frozen spermatozoa are thawed in an embryo culture medium.

On the other hand, the diluent for human frozen spermatozoa used in the methods of the present invention has a pH of 6.9 to 7.1.

As described in the Examples below, in cases where the diluent for human frozen spermatozoa has a pH of 7.0, sperm motility is still high 24 hours after thawing of human frozen spermatozoa. In artificial insemination, a spermatozoon is required to reach an ovum at the time of ovulation to achieve fertilization, and the process takes a certain period of time. Therefore, the spermatozoon is required to maintain high motility until the time of fertilization. In cases where human frozen spermatozoa are thawed and mixed in a diluent for human frozen spermatozoa at pH 7.0, the spermatozoa maintain sperm motility even 24 hours later, so that the diluent is useful also in cases of artificial insemination and in vitro fertilization, and it is possible to expect an improved pregnancy rate in artificial insemination and the like.

The diluent for human frozen spermatozoa described above is obtained by adding a chelating agent that forms a complex with a calcium ion to a basic diluent.

The basic diluent herein is a diluent prepared such that the diluent allows collected semen to maintain the function of spermatozoa contained therein at room temperature for a predetermined period of time, and is a liquid containing components such as glucose, sodium citrate, sodium bicarbonate, EDTA-2Na, citric acid, Tris and potassium chloride. The basic diluent is not limited as long as the basic diluent is a liquid normally used in the field. Examples of the basic diluent include Modena solution (0.15 M glucose, 26.7 mM sodium citrate, 11.9 mM sodium hydrogen carbonate, 15.1 mM citric acid, 6.3 mM EDTA-2Na, 46.6 mM Tris and 1,000 IU/ml penicillin).

The chelating agent comprises ethylene glycol tetraacetic acid (EGTA (ethylene glycol bis(2-aminoethyl)-N,N,N',N'-tetraacetic acid)). EGTA effectively prevents calcium ions from acting on mitochondria of spermatozoa by specifically forming a complex with a calcium ion.

Further, in addition to EGTA, ethylenediaminetetraacetic acid (EDTA) is contained. EDTA is a substance that forms complexes with various divalent ions such as a magnesium ion and zinc ion, in addition to a calcium ion.

When both EDTA and EGTA are contained, calcium ions which were not chelated by EGTA are chelated, and most calcium ions present in the basic diluent are eventually chelated. For example, in cases where only EDTA is used as the chelating agent, chelating of all calcium ions with EDTA is impossible without increasing the concentration of EDTA in the basic diluent. The basic diluent contains various divalent ions other than calcium ions, such as magnesium ions and zinc ions. In cases where the EDTA concentration is too high, there is the risk of overchelation of such divalent ions, which influences the sperm function (fertilization). Therefore, the diluent for human frozen spermatozoa contains both EDTA and EGTA.

Further, by adjusting the pH, the diluent for human frozen spermatozoa is obtained. For example, addition of a chelating agent such as EGTA to the above-mentioned Modena solution (pH 7.4) lowers the pH. Therefore, for example, the pH is adjusted to a value from 6.9 to 7.4 by addition of an alkaline solution such as an aqueous NaOH solution.

### (Method for Preparing Semen for In Vitro Fertilization or Semen for Artificial Insemination in Humans)

A method for diluting human frozen spermatozoa, and a method for preparing semen for in vitro fertilization and the like are described below in detail.

For example, the human frozen spermatozoa to be used are frozen spermatozoa prepared by carrying out freezing as follows. Collected semen is diluted in a diluent such as Modena solution, and liquefied for not less than 15 minutes after ejaculation, followed by separation of spermatozoa from seminal plasma. The spermatozoa are resuspended in a diluent such as Modena solution, and the temperature is lowered to about 15°C. The spermatozoa are then separated from the diluent. For example, the separation is carried out by centrifugation or the like.

To the separated spermatozoa, a hyperosmotic liquid is added, and the resulting mixture is mixed. The temperature of the mixture is then lowered to about 5°C for about 2 hours. By such hyperosmotic treatment, water molecules in the spermatozoa are removed. The treatment is carried out because the volume of water molecules increases in the freezing process and the increase in the volume causes a stress to the spermatozoa. For example, the hyperosmotic liquid to be used is a solution prepared by addition of a sugar such as sucrose to an osmotic pressure of more than 300 mOsm/kg and less than 500 mOsm/kg. In cases where the osmotic pressure is not more than 300 mOsm/kg, dehydration is insufficient, and an increase in the volume of water molecules during freezing causes a stress to the spermatozoa. Further, in cases where the osmotic pressure is not less than 500 mOsm/kg, water in the spermatozoa is also removed, causing death of the spermatozoa. A hyperosmotic liquid with an osmotic pressure of 400 mOsm/kg is more preferably used.

The spermatozoa are preferably frozen after the hyperosmotic treatment and, further, addition of a cryoprotective agent. Examples of the cryoprotective agent include glycerol. More specifically, a solution containing glycerol is further added to the above hyperosmotic liquid, to perform glycerol equilibration. The addition is carried out such that the glycerol concentration is adjusted to a level higher than 2% by volume and lower than 5% by volume, and the glycerol equilibration is then carried out for about 20 minutes. A predetermined container is filled with the mixture after glycerol equilibration, and the mixture is then frozen. In cases where the glycerol concentration is not more than 2 v/v%, the cell membrane of the spermatozoa is deteriorated. Further, in cases where the glycerol concentration is not less than 5 v/v%, the motility after thawing is low. The glycerol concentration is preferably adjusted to 3 v/v%.

The freezing method is not limited as long as freezing of spermatozoa is possible therewith, and examples of the method include a method wherein spermatozoa are frozen by immersion in liquid nitrogen. For example, in cases where spermatozoa are to be frozen in liquid nitrogen, the spermatozoa are not immediately immersed in the liquid nitrogen. A container filled with spermatozoa is fixed at a position slightly distant from the surface of the liquid nitrogen to perform prefreezing for a predetermined period, and the container is then immersed in the liquid nitrogen to perform freezing and storing.

The thus frozen and stored human spermatozoa are thawed, and the thawed spermatozoa are quickly mixed with the diluent for human frozen spermatozoa. Various methods are available for thawing the human frozen spermatozoa, and examples of the methods include a method wherein a container filled with human frozen spermatozoa is immersed in warm water at about 37°C for about 60 seconds. Thereafter, the thawed spermatozoa are added to a diluent for human frozen spermatozoa whose temperature was adjusted to about 37°C, and the resulting mixture is mixed. When the spermatozoa are mixed with the diluent for human frozen spermatozoa, a chelating agent contained in the diluent for human frozen spermatozoa is bound to calcium ions to form complexes, resulting in suppression of the action of the calcium ions on the spermatozoa. Therefore, vigorous movement of the spermatozoa, which leads to death of the spermatozoa, is suppressed.

For example, after the start of thawing, the thawed spermatozoa are separated from the diluent for human frozen spermatozoa within a predetermined period of time. By the separation, the spermatozoa are separated from the chelating agents comprising EDTA and EGTA. While the drastic action of calcium ions during thawing/immediately after thawing of the frozen spermatozoa needs to be suppressed, calcium ions are also an indispensable component for survival of spermatozoa. After thawing of human frozen spermatozoa, death of the spermatozoa occurs if the spermatozoa are continuously kept under conditions where incorporation of calcium ions is impossible for a long time.

After separation of the spermatozoa from the chelating agents comprising EDTA and EGTA, the spermatozoa has an ability to incorporate calcium ions, so that survival of the spermatozoa is secured. The separation of spermatozoa from the diluent for human frozen spermatozoa is preferably carried out immediately after sufficient mixing, and is preferably carried out within 15 minutes after the start of thawing. The separation of spermatozoa from the diluent for human frozen spermatozoa is carried out by centrifugation or the like. In cases where centrifugation is carried out to separate the spermatozoa from the diluent for human frozen spermatozoa, the centrifugation is carried out at a rotation speed that does not cause a stress on the spermatozoa. In such cases, centrifugation at 400 G for about 10 minutes allows the separation. It should be noted that, as described in Examples below, Patent Literature 1 discloses thawing, diluting and culturing of pig frozen spermatozoa wherein culturing is directly carried out without separation of the chelating agent, and high motility of the pig spermatozoa is maintained thereafter. However, human spermatozoa die due to lack of calcium in cases where the human spermatozoa are not separated from the chelating agent within a predetermined period of time after the start of thawing.

For example, the separated spermatozoa is transferred to an embryo culture medium and cultured in preparation for artificial insemination or in vitro fertilization. The embryo culture medium is a medium containing components necessary for survival of spermatozoa, and is not limited as long as the embryo culture medium is one normally used in the field.

The term "in vitro fertilization" herein means performing fertilization outside the body of human, and the term more specifically means adding semen dropwise onto an ovum removed from human to cause fertilization, or directly injecting a spermatozoon selected from semen into an ovum under the microscope. On the other hand, the term "artificial insemination" means injecting spermatozoa which were separately collected, or semen prepared after collection, into the body of human.

After thawing of frozen spermatozoa, it is preferred to carry out freezing using a straw for freezing (cylindrical body) such that the thawed spermatozoa and the diluent for human frozen spermatozoa are immediately and sufficiently mixed together. For example, a straw for freezing is filled with the spermatozoa and the diluent for human frozen spermatozoa, and the spermatozoa and the diluent are frozen in the straw. For example, in order to prevent mixing of the spermatozoa with the diluent for human frozen spermatozoa before thawing, the spermatozoa and the diluent for human frozen spermatozoa are fed to, enclosed in, and frozen in the straw such that an air layer is placed therebetween.

By immersing the frozen straw in a warm bath for a predetermined period of time, the spermatozoa and the diluent for human frozen spermatozoa in the straw are thawed, and the spermatozoa are quickly mixed with the diluent for human frozen spermatozoa. Therefore, the action of calcium ions on the spermatozoa is quickly suppressed.

### (Test for Sperm Motility after Thawing under Influence of Hyperosmotic Treatment)

The collected semen was separated into spermatozoa and seminal plasma, and the separated spermatozoa were treated with a hyperosmotic liquid and frozen. The frozen spermatozoa were then thawed and subjected to a test for the sperm motility after thawing.

### Comparative example 1a (using Medium 199 as diluent)

The whole amount of semen collected from human was transferred into a centrifuge tube (Falcon conical tube 2095) with a dropper (Falcon pipette 7575), and the amount, the state of liquefaction and the presence/absence of jelly were investigated.

The semen was diluted in Modena solution (0.15 M glucose, 26.7 mM sodium citrate, 11.9 mM sodium hydrogen carbonate, 15.1 mM citric acid, 6.3 mM EDTA-2Na, 46.6 mM Tris and 1,000 U/ml penicillin), and liquefied for not less than 15 minutes after ejaculation. Thereafter, the seminal plasma and Modena solution were removed by centrifugation (400 G, 10 minutes), and the separated spermatozoa were resuspended in Modena solution. The temperature was lowered to 15°C.

Modena solution was removed by centrifugation (400 G, 10 minutes), to separate spermatozoa.

At 15°C, diluent 1 in the same amount as the separated spermatozoa was added to the separated spermatozoa, and the resulting mixture was sufficiently mixed. Thereafter, the mixture was cooled to 5°C for 2 hours. The diluent 1 used herein was prepared by preparing a sucrose solution such that the osmotic pressure of the solution is 400 mOsm/kg and adding egg yolk thereto at 20%. By hyperosmotic treatment using the hyperosmotic liquid with an osmotic pressure of 400 mOsm/kg, the spermatozoa were dehydrated to an appropriate level.

Subsequently, at 5°C, diluent 2 in the same amount as the mixture of diluent 1 and spermatozoa was added to the mixture, and the resulting mixture was mixed. Thereafter, glycerol equilibration was carried out for 20 minutes to dehydrate the spermatozoa. The diluent 2 used herein was prepared by mixing OEP (Orvus ES Paste) (0.15 mL) as a surfactant and glycerol (0.6 mL) as a cryoprotective agent with the above-described diluent 1 (9.25 mL), to prepare a solution having a glycerol concentration of 3% (v/v). The prepared diluent 2 was also used after lowering the liquid temperature to 5°C.

The thus prepared mixture of the spermatozoa and the diluent was aliquoted in appropriate volumes into straws for freezing. The straws were then fixed at positions 4 cm distant from the liquid surface of liquid nitrogen, and left to stand in the vapor for 7 minutes, to carry out prefreezing. Thereafter, the straws were immersed in liquid nitrogen, to carry out cryopreservation.

The frozen straws were removed from liquid nitrogen, and immersed in warm water at 37°C for 5 to 10 seconds for thawing. The whole content in each straw was transferred into a centrifuge tube, and a medium was added thereto, followed by centrifugation (400 G, 10 minutes). Thereafter, the supernatant was removed, and the medium was added again to the tube, followed by centrifugation (400 G, 10 minutes). Thereafter, the supernatant was removed, and the medium was added to the tube to adjust the volume to 1 mL, followed by performing culture. The medium was Medium 199 (GIBCO 11150) supplemented with 10% (v/v) Serum Substitute Supplement (1. S. Japan).

Immediately after thawing and 6 hours after thawing, the motility rate of spermatozoa (sperm motility) was measured. The sperm motility was measured using a Makler chamber (Sefi-Medical Instrument). Onto a Makler chamber, 20 µL of the sample was added dropwise, and counting was performed for 10 to 50 squares each at not less than 3 positions, such that the number of spermatozoa counted was not less than 200. The sperm motility is the ratio of spermatozoa having motility, and spermatozoa having forward motility were regarded as having motility.

### (Comparative Example 1)

Further, as a Comparative Example, freezing and thawing of spermatozoa were carried out by a conventional method. More specifically, as described below, using a commercially available solution for freezing spermatozoa, preparation of frozen spermatozoa and thawing of frozen spermatozoa were carried out according to the protocols attached to the commercially available solution, and the sperm motility after thawing was measured.

The collected semen was left to stand until the semen was liquefied, and the whole amount of semen was transferred into a centrifuge tube (Falcon conical tube 2095) with a pipette (Falcon pipette 7575), followed by investigation of the amount, the state of liquefaction and the presence/absence of jelly.

A medium was added to the semen, and the resulting mixture was mixed and centrifuged (200 G, 10 minutes). The medium was Medium 199 (GIBCO 11150) supplemented with 10% (v/v) Serum Substitute Supplement (I. S. Japan).

Thereafter, the supernatant was removed to obtain a pellet. The supernatant was mixed and centrifuged (200 G, 10 minutes).

Further, the supernatant was removed, and a fresh medium was added to the pellet to adjust the volume to 0.3 mL.

The semen whose volume was adjusted to 0.3 mL was transferred into a 0.5-mL vial, and an equal amount (0.5 mL) of Freezing Medium-TEST Yolk Buffer with Glycerol as a medium was added dropwise thereto such that the ratio between the medium and the semen became 1:1 (v:v). The composition of the Freezing Medium-TEST Yolk Buffer with Glycerol employed is described below.

### TES (N-tris(hydroxymethyl)-methyl-2-aminoethanesulfonic acid)

### Tris (tris(hydroxymethyl amino ethane)

### Sodium citrate

### Fructose

| | |
|---|---|
| Gentamycin | 10 µg/mL |
| Glycerol | 12% (v/v) |
| Egg yolk | 20%(v/v) (inactivated) |

The mixture of the medium and the semen was slowly cooled from 37°C to 5°C (-0.5°C/min.). Ninety minutes after the start of cooling, the mixture was aliquoted in appropriate amounts into freezing canes.

Thereafter, the canes were fixed at positions 4 cm distant from the liquid surface of liquid nitrogen, and left to stand in the vapor for 7 minutes, to carry out prefreezing. Thereafter, the canes were immersed in liquid nitrogen, to carry out cryopreservation.

In the same manner as in Comparative example 1a, the frozen canes were removed from liquid nitrogen, and immersed in warm water at 37°C for 5 minutes for thawing. The whole content in each cane was transferred into a centrifuge tube using a pipette, and the above-described medium was added thereto, followed by centrifugation (400 G, 10 minutes). Thereafter, the supernatant was removed, and the medium was added again to the tube, followed by centrifugation (400 G, 10 minutes). The supernatant was then removed, and the medium was added to the tube to adjust the volume to 1 mL, followed by performing culture.

Using a Makler chamber as in Comparative example 1a, the sperm motility was measured immediately after thawing and 6 hours after thawing.

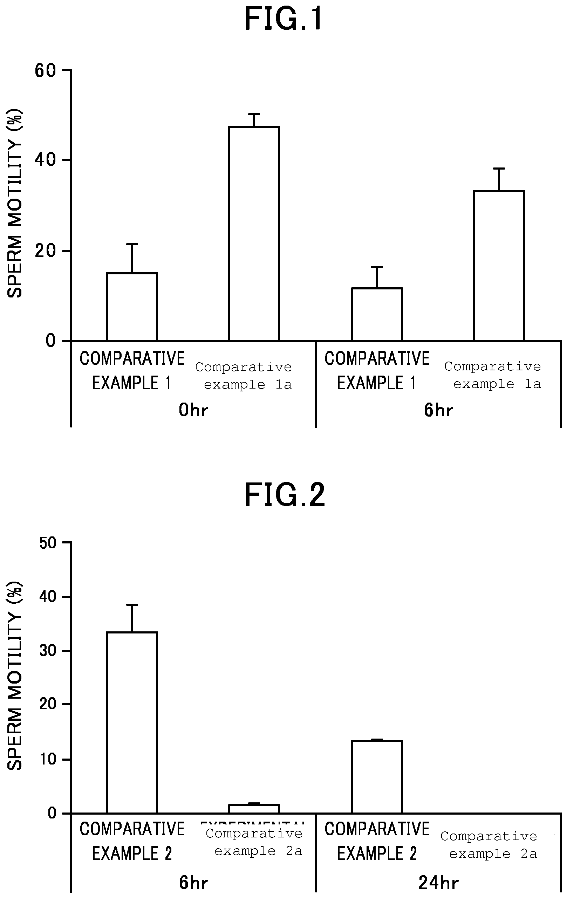

FIG. 1 shows the results of measurement of the sperm motility of spermatozoa after thawing in each of Comparative example 1a and Comparative Example 1.

The results obtained immediately after thawing and 6 hours after thawing both showed that Comparative example 1a, wherein thawing was carried out using the diluent Medium 199 resulted in higher sperm motility than Comparative Example 1. It is thought that since, in Comparative example 1, spermatozoa prepared by removal of seminal plasma from semen were subjected to hyperosmotic treatment, the stress caused on the spermatozoa due to freezing was reduced, resulting in the high motility after thawing.

### (Test for Motility of Spermatozoa Prepared by Thawing and Culturing Human Frozen Spermatozoa Using Diluent and Dilution Method of Patent Literature 1)

Subsequently, human frozen spermatozoa were thawed and cultured using the diluent and the method described in Patent Literature 1, and the sperm motility after thawing was tested. The diluent and the method of Patent Literature 1 are a diluent and a method of thawing and culturing for artificial insemination in mainly polytocous animals such as pig (the diluent is hereinafter referred to as "diluent for pig frozen spermatozoa").

### Comparative example 2a (pH=7.4)

To a straw for freezing, 50 µL of a mixture of the spermatozoa and the diluent prepared in the same manner as in Example 1 was fed, and 50 µL of a diluent for pig frozen spermatozoa was further fed thereto such that an air layer separates the mixture from the diluent. The straw was then sealed.

The diluent for pig frozen spermatozoa employed was a liquid prepared by adding EGTA to the above-mentioned Modena solution and adjusting the pH by dropwise addition of NaOH, wherein the final concentration of EGTA was 6 mM and the pH was 7.4.

The straw filled with the above-described mixture and diluent for pig frozen spermatozoa and sealed was placed at a position 4 cm distant from the upper surface of liquid nitrogen, and prefreezing was carried out for 7 minutes. The straw was then stored in liquid nitrogen (-196°C).

The frozen straw was removed from liquid nitrogen, and immersed in warm water at 37 to 40°C for 5 to 10 seconds for thawing.

The content of the straw was transferred into a 15-mL centrifuge tube (Falcon conical tube 2095) such that the thawed spermatozoa were immediately mixed with the diluent for pig frozen spermatozoa, and the thawed spermatozoa were cultured directly.

Using a Makler chamber as in Comparative example 1a, the sperm motility was measured
6 hours after thawing and 24 hours after thawing.

### (Comparative Example 2)

Further, as a Comparative Example, human frozen spermatozoa were thawed and cultured in the same manner as in Comparative example 2a except that an embryo culture medium (solution prepared by adding SSS (Serum Substitute Supplement; manufactured by I. S. Japan) to HFF99 (manufactured by FUSO Pharmaceutical Industries, Ltd.) such that 10% (v/v) SSS is contained) was used instead of the diluent for pig frozen spermatozoa.

Thereafter, using a Makler chamber as in Comparative example 1a, the sperm motility was measured 6 hours after thawing and 24 hours after thawing.

FIG. 2 shows the results of measurement of the sperm motility after thawing in Comparative example 2a and Comparative Example 2.

The motility of the spermatozoa thawed and cultured with the diluent for pig frozen spermatozoa of Comparative example 2a was lower than the motility of the spermatozoa thawed and cultured with the solution of Comparative Example 2 prepared by adding SSS to HFF99, in both the result obtained 6 hours after thawing and the result obtained 24 hours after thawing. It was therefore found that spermatozoa die under conditions where calcium ions do not act on the spermatozoa for a long time after thawing. It was found that direct application of the method for thawing pig frozen spermatozoa of Patent Literature 1 to human frozen spermatozoa does not allow maintenance of the sperm motility.

### (Test for Motility of Spermatozoa Prepared by Thawing Frozen Spermatozoa, Mixing Thawed Spermatozoa with Diluent for Human Frozen Spermatozoa, and Separating Spermatozoa from Diluent for Human Frozen Spermatozoa)

Subsequently, the motility of spermatozoa prepared by thawing human frozen spermatozoa, mixing the thawed spermatozoa with a diluent for human frozen spermatozoa, and separating the spermatozoa from the diluent for human frozen spermatozoa was tested.

### (Example 2)

First, EGTA was added to Modena solution, and NaOH was added dropwise to the resulting mixture to prepare a diluent for human frozen spermatozoa having a pH of 7.0. The final concentration of EGTA in the diluent for human frozen spermatozoa was 6 mM.

Thereafter, in the same manner as in Comparative example 1a, a straw for freezing was filled with the mixture of the spermatozoa and the diluent, and frozen.

The frozen straw was removed from liquid nitrogen, and immersed in warm water at 37 to 40°C for 60 seconds, for thawing. The content of the straw was then transferred into a 15-mL centrifuge tube containing the diluent for human frozen spermatozoa prewarmed to 37°C.

Thereafter, centrifugation (400 G, 10 minutes) was immediately carried out to separate the spermatozoa from the diluent for human frozen spermatozoa. The supernatant was then removed, and the separated spermatozoa were cultured in an embryo culture medium. The embryo culture medium was a medium prepared by adding SSS (Serum Substitute Supplement; manufactured by I. S. Japan) to HFF99 (manufactured by FUSO Pharmaceutical Industries, Ltd.) such that 10% (v/v) SSS was contained.

The sperm motility was measured 15 minutes, 30 minutes, 60 minutes and 120 minutes after the thawing of frozen spermatozoa. The measurement of sperm motility was carried out in the same manner as in Comparative example 1a.

### (Comparative Example 3)

Further, frozen spermatozoa were thawed and cultured in the same manner as in Example 2 except that the spermatozoa were not separated from the diluent for human frozen spermatozoa, and the sperm motility was measured.

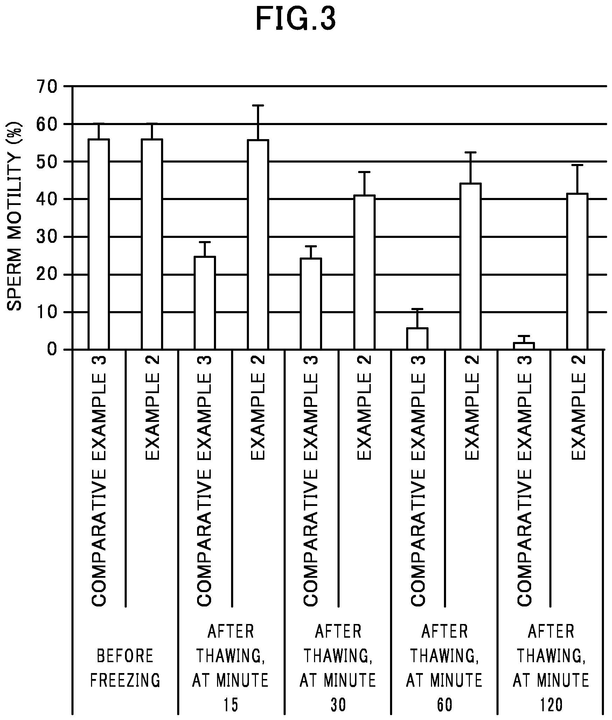

The results of Example 2 and Comparative Example 3 are shown in FIG. 3. In Example 2, wherein the spermatozoa were separated from the diluent for human frozen spermatozoa, the motility observed 15 minutes after thawing was almost the same as the motility observed before freezing, and the motility was kept high even 30 minutes, 60 minutes and 120 minutes after thawing. On the other hand, in Comparative Example 3, wherein the spermatozoa were not separated from the diluent for human frozen spermatozoa, the motility decreased by half by 15 minutes after thawing compared to the motility observed before freezing. The motility further continued to decrease thereafter, and was almost lost 120 minutes after thawing.

Based on the above results, it was found that, by thawing human frozen spermatozoa, mixing the thawed spermatozoa with the diluent for human frozen spermatozoa, and then immediately separating the spermatozoa from the diluent for human frozen spermatozoa, the sperm motility is kept high thereafter. Further, since, in the case where the spermatozoa were not separated from the diluent for human frozen spermatozoa, the sperm motility decreased by half by 15 minutes after thawing compared to the sperm motility observed before freezing, it is thought that separation of spermatozoa from the diluent for human frozen spermatozoa within 15 minutes after the start of thawing is preferred.

### (Test for Influence ofpH of Diluent for Human Frozen Spermatozoa on Sperm Motility)

Subsequently, diluents for human frozen spermatozoa having different pHs were adjusted, and thawed human frozen spermatozoa were mixed with the diluents for human frozen spermatozoa. The spermatozoa were then separated from the diluents for human frozen spermatozoa and cultured, followed by measurement of the sperm motility after thawing, in order to test the influence of the pH of the diluent for human frozen spermatozoa on sperm motility.

### (Example 3)

First, diluents for human frozen spermatozoa having different pHs were prepared. EGTA was added the above-described Modena solution, and NaOH was added dropwise thereto to adjust the pH, to prepare diluents for human frozen spermatozoa having 4 different pHs, 7.0 (according to the invention) and for comparison 6.5, 6.75 and 7.4. The final concentration of EGTA was 6 mM in all cases.

Thereafter, using the prepared diluents for human frozen spermatozoa having different pHs, human frozen spermatozoa were thawed, diluted and cultured by the same method as in Example 2. Subsequently, as in Comparative example 1a, the sperm motility was measured using a Makler chamber 6 hours after thawing and 24 hours after thawing.

### (Comparative Example 4)

Further, as a Comparative Example, human frozen spermatozoa were thawed and cultured by the same method as in Comparative Example 2 without using the diluent for human frozen spermatozoa. The sperm motility was then measured as in Comparative example 1a using a Makler chamber 6 hours after thawing and 24 hours after thawing.

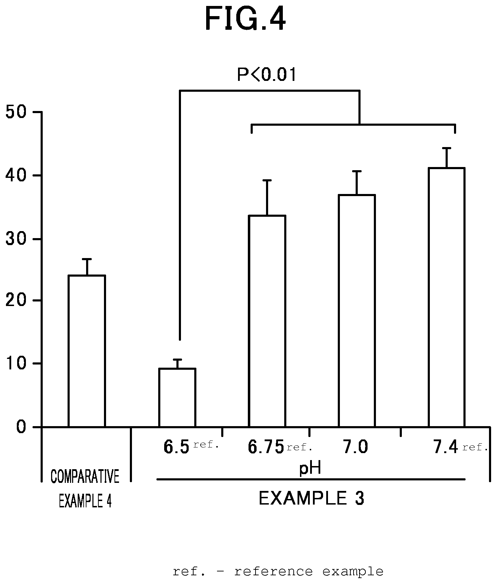

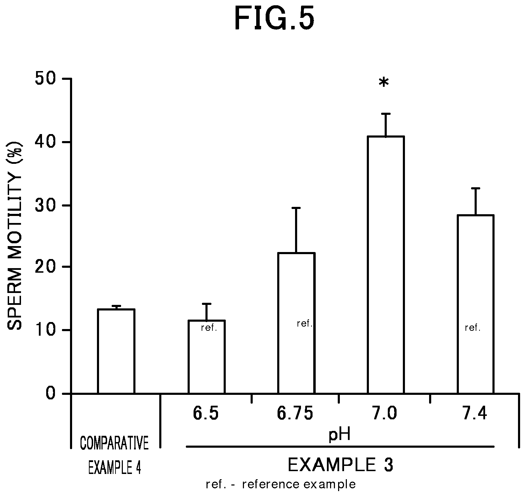

In terms of the results of Example 3 and Comparative Example 4, the sperm motility observed 6 hours after thawing and the sperm motility observed 24 hours after thawing are shown in FIG. 4 and FIG. 5, respectively.

In Example 3, when the diluents for human frozen spermatozoa having pHs of 6.75, 7.0 and 7.4 were used, the sperm motility was significantly higher in both the result obtained 6 hours after thawing and the result obtained 24 hours after thawing, compared to the case where HFF was used for thawing and culturing of frozen spermatozoa (Comparative Example 4).

Further, when the diluent for human frozen spermatozoa having a pH of 7.0 was used, the sperm motility was kept high even 24 hours after thawing, and the best result was obtained. It is thought that the diluent for human frozen spermatozoa having a pH of about 7.0 (pH of about 6.9 to 7.1) allows maintenance of high sperm motility for a long time after thawing.

### (Test for Fertility)

Further, human frozen spermatozoa were thawed and mixed with the diluent for human frozen spermatozoa, and the spermatozoa were then separated from the diluent for human frozen spermatozoa and cultured. Whether the spermatozoa have fertility or not was then tested.

Using spermatozoa from the same subjects, frozen spermatozoa were prepared in the same manner as in Experimental Example, and the frozen spermatozoa were subjected to the following Example 4 and Comparative Example 5. The test was performed for two subjects (Patient number 1,2).

### (Example 4)

In the same manner as in Example 2, human frozen spermatozoa were thawed and mixed with the diluent for human frozen spermatozoa, and spermatozoa were then separated from the diluent for human frozen spermatozoa. The pH of the diluent for human frozen spermatozoa was 7, and the final concentration of EGTA was 6 mM.

The separated spermatozoa were cultured in an embryo culture medium supplemented with 4 mM caffein (Caf) or a caffeine-free embryo culture medium (free).

After 60 minutes of culture, spermatozoa were collected by centrifugation, and dissolved in SDS sample buffer (SDS (sodium dodecyl sulfate) buffer). Thereafter, the resultant was subjected to 10% SDS-PAGE (SDS (sodium dodecyl sulfate)-polyacrylamide gel electrophoresis) and transferred onto a PVDF membrane (polyvinylidene fluoride membrane), followed by detection of the level of phosphorylation of tyrosine residues in the proteins of spermatozoa using an anti-Tyr phosphorylation antibody.

### (Comparative Example 5)

In the same manner as in Comparative Example 2, human frozen spermatozoa were thawed and cultured. The level of phosphorylation of tyrosine residues in the proteins of spermatozoa was detected in the same manner as in Example 4.

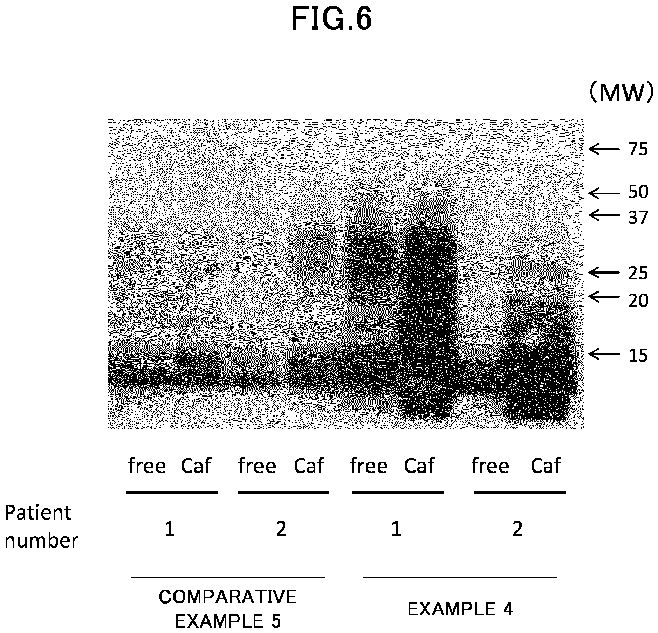

The results of detection of bands indicating phosphorylation of tyrosine residues in Example 4 and Comparative Example 5 are shown in FIG. 6. Whether or not phosphorylation of tyrosine residues in the proteins of spermatozoa occurs is used as an index for judging whether or not the spermatozoa have fertility. Since tyrosine residues in the proteins are phosphorylated by mediation of caffeine, detection of the phosphorylation bands allows confirmation of the fact that the spermatozoa have fertility.

In Comparative Example 5, only faint tyrosine phosphorylation bands appeared in each of free and Caf, indicating that the spermatozoa of Comparative Example 5 do not have much fertility. On the other hand, in both subjects in Example 4, Caf showed tyrosine phosphorylation bands which are more intense than the bands in free, indicating that the spermatozoa of Example 4 have sufficient fertility.

Thus, after thawing of human frozen spermatozoa, the thawed spermatozoa maintain high motility and sufficient fertility for a long time, so that an improved pregnancy rate is expected not only in in vitro fertilization but also in artificial insemination.

### Industrial Applicability

The diluent for human frozen spermatozoa used in the methods of the present invention contains EDTA and EGTA that forms a complex with a calcium ion, and has a pH of 6.9 to 7.1.

The action of a large amount of calcium ions on spermatozoa is suppressed during thawing and immediately after thawing of human frozen spermatozoa. Therefore, vigorous movement of the spermatozoa by the action of calcium ions, which leads to death of the spermatozoa, during thawing and immediately after thawing is suppressed, and sperm motility is maintained for a long time after thawing. Therefore, the diluent is applicable to artificial insemination, in vitro fertilization and the like, and improvement of the pregnancy rate is expected.

Further, in the method for preparing semen for in vitro fertilization or semen for artificial insemination in humans, human frozen spermatozoa are thawed and mixed with the diluent for human frozen spermatozoa. Thereafter, by separating the thawed spermatozoa from the diluent for human frozen spermatozoa within a predetermined period of time after the start of thawing of human frozen spermatozoa, semen for human artificial insemination or the like is obtained. By suppressing the drastic action of calcium ions on spermatozoa during thawing and immediately after thawing, and by separating the thawed spermatozoa from the diluent for human frozen spermatozoa after mixing, supplying of calcium, which is required thereafter for the activity of spermatozoa, is possible. In the thus obtained semen for human artificial insemination the activity of spermatozoa is maintained for a long time, so that the semen is applicable to artificial insemination.

## Claims

1. A method for preparing semen for in vitro fertilization or semen for artificial insemination in humans, wherein human frozen spermatozoa are thawed and mixed with a diluent for human frozen spermatozoa, and spermatozoa are then separated from the diluent for human frozen spermatozoa, the diluent for human frozen spermatozoa, comprising a chelating agent that forms a complex with a calcium ion, and having a pH of 6.9 to 7.1, said chelating agent comprises EGTA and EDTA.

2. The method for preparing semen for in vitro fertilization or semen for artificial insemination in humans according to Claim 1, wherein the spermatozoa are separated within 15 minutes after the start of thawing.

3. The method for preparing semen for in vitro fertilization or semen for artificial insemination in humans according to Claims 1 or 2, wherein the separated spermatozoa are cultured in an embryo culture medium.

4. The method for preparing semen for in vitro fertilization or semen for artificial insemination in humans according to any one of Claims 1 to 3, wherein the human frozen spermatozoa are prepared by separating human semen into spermatozoa and seminal plasma, dehydrating the separated spermatozoa by hyperosmotic treatment, and freezing the dehydrated spermatozoa.

5. The method for preparing semen for in vitro fertilization or semen for artificial insemination in humans according to Claim 4, wherein a hyperosmotic liquid with an osmotic pressure of more than 300 mOsm/kg and less than 500 mOsm/kg is used.

6. The method for preparing semen for in vitro fertilization or semen for artificial insemination in humans according to Claim 4 or 5, wherein the human frozen spermatozoa are prepared by adding glycerol and performing glycerol equilibration under conditions where the glycerol concentration is higher than 2% by volume and lower than 5% by volume, followed by freezing.

## Patentansprüche

1. Verfahren zur Präparation von Spermien für die In-vitro-Fertilisation oder Spermien für die künstliche Insemination bei Menschen, wobei menschliche, gefrorene Spermatozoen aufgetaut und mit einem Verdünnungsmittel für menschliche, gefrorene Spermatozoen gemischt werden und die Spermatozoen anschließend vom Verdünnungsmittel für menschliche, gefrorene Spermatozoen getrennt werden, wobei das Verdünnungsmittel für menschliche, gefrorene Spermatozoen einen Chelatbildner umfasst, der einen Komplex mit einem Calciumion bildet, und einen pH-Wert von 6,9 bis 7,1 aufweist, und wobei der Chelatbildner EGTA und EDTA umfasst.

2. Verfahren zur Präparation von Spermien für die In-vitro-Fertilisation oder Spermien für die künstliche Insemination bei Menschen nach Anspruch 1, wobei die Spermatozoen innerhalb von fünfzehn Minuten nach dem Beginn des Auftauens getrennt werden.

3. Verfahren zur Präparation von Spermien für die In-vitro- Fertilisation oder Spermien für die künstliche Insemination bei Menschen nach Anspruch 1 oder 2, wobei die getrennten Spermatozoen in einem Embryo-Kulturmedium kultiviert werden.

4. Verfahren zur Präparation von Spermien für die In-vitro- Fertilisation oder Spermien für die künstliche Insemination bei Menschen nach einem der Ansprüche 1 bis 3, wobei die menschlichen, gefrorenen Spermatozoen zubereitet werden, indem menschliche Spermien in Spermatozoen und Samenplasma getrennt, die getrennten Spermatozoen in einer hyperosmotischen Behandlung dehydriert und die dehydrierten Spermatozoen eingefroren werden.

5. Verfahren zur Präparation von Spermien für die In-vitro-Fertilisation oder Spermien für die künstliche Insemination bei Menschen nach Anspruch 4, wobei eine hyperosmotische Flüssigkeit mit einem osmotischen Druck von über 300 mOsm/kg und weniger als 500 mOsm/kg verwendet wird.

6. Verfahren zur Präparation von Spermien für die In-vitro-Fertilisation oder Spermien für die künstliche Insemination bei Menschen nach Anspruch 4 oder 5, wobei die menschlichen, gefrorenen Spermatozoen durch Hinzufügen von Glycerol und Durchführen einer Glycerol-Äquilibrierung unter Bedingungen, bei denen die Glycerolkonzentration höher als 2 Volumenprozent und geringer als 5 Volumenprozent ist, gefolgt von Einfrieren, zubereitet werden.

## Revendications

1. Un procédé pour préparer du sperme pour la fécondation in vitro ou du sperme pour l'insémination artificielle chez les humains, dans lequel les spermatozoïdes humains congelés sont décongelés et mélangés avec un diluant pour des spermatozoïdes humains congelés, et ensuite les spermatozoïdes sont séparés du diluant pour des spermatozoïdes humains congelés, le diluant pour des spermatozoïdes humains comprenant un agent chélateur qui forme un complexe avec un ion calcium, et ayant un pH qui va de 6,9 à 7,1; ledit agent chélateur comprend de l'EGTA et de l'EDTA.

2. Le procédé pour préparer du sperme pour la fécondation in vitro ou du sperme pour l'insémination artificielle chez les humains selon la revendication 1, dans lequel les spermatozoïdes sont séparés dans les 15 minutes qui suivent le début de la décongélation.

3. Le procédé pour préparer du sperme pour la fécondation in vitro ou du sperme pour l'insémination artificielle chez les humains selon les revendications 1 ou 2, dans lequel les spermatozoïdes séparés sont mis en culture dans un milieu de culture d'embryons.

4. Le procédé pour préparer du sperme pour la fécondation in vitro ou du sperme pour l'insémination artificielle chez les humains selon l'une quelconque des revendications 1 à 3, dans lequel les spermatozoïdes humains congelés sont préparés en séparant le sperme humain en spermatozoïdes et plasma séminal, en déshydratant les spermatozoïdes séparés à l'aide d'un traitement hyperosmotique, et en congelant les spermatozoïdes déshydratés.

5. Le procédé pour préparer du sperme pour la fécondation in vitro ou du sperme pour l'insémination artificielle chez les humains selon la revendication 4, dans lequel l'on utilise un liquide hyperosmotique ayant une pression osmotique supérieure à 300 mOsm/kg et inférieure à 500 mOsm/kg.

6. Le procédé pour préparer du sperme pour la fécondation in vitro ou du sperme pour l'insémination artificielle chez les humains selon les revendications 4 ou 5, dans lequel les spermatozoïdes humains congelés sont préparés en ajoutant du glycérol et en exécutant une stabilisation du glycérol dans des conditions où la concentration de glycérol est supérieure à 2% en volume et inférieure à 5% en volume, ce qui est suivit de la congélation.
